# EUROPÄISCHE PATENTSCHRIFT

(11) **EP 1 793 744 B1**
(45) Veröffentlichungstag und Bekanntmachung des Hinweises auf die Patenterteilung: **17.12.2008**
(21) Anmeldenummer: 04786980.5
(22) Anmeldetag: 22.09.2004
(51) Int. Cl.: A61B 17/12

(54) **MEDIZINISCHES IMPLANTAT**
MEDICAL IMPLANT
IMPLANT MEDICAL

(43) Veröffentlichungstag der Anmeldung: 13.06.2007
(73) Patentinhaber: Dendron GmbH, 44799 Bochum (DE)
(72) Erfinder: MONSTADT, Hermann, 44797 Bochum (DE); HENKES, Hans, 44797 Bochum (DE); FLESSER, Achim, 40822 Mettmann (DE); HANNES, Ralf, 44137 Dortmund (DE)
(74) Vertreter: Thiel, Christian
(86) Internationale Anmeldenummer: PCT/EP2004/010610
(87) Internationale Veröffentlichungsnummer: WO 2006/032289

(56) Entgegenhaltungen:
- WO-A-01/93937
- WO-A-03/017852
- WO-A-03/041615
- US-A1- 2002 019 647
- US-A1- 2004 045 554
- US-B1- 6 280 457

## Beschreibung

Die Erfindung betrifft ein medizinisches Implantat in Form einer langgestreckten Wendel, wobei zumindest ein Teil der Wendel zur Einnahme einer Sekundärstruktur vorgeformt ist, die sie am Implantationsort während der Implantation einnimmt, und die ihrerseits am Implantationsort während der Implantation eine polyederförmige Tertiärstruktur ausbildet, wobei jede Fläche des Polyeders von einer Schlinge gebildet wird.

Die Erfindung betrifft weiterhin eine Vorrichtung zur Implantation solcher Implantate in Körperhohlräume und -gefäßen.

Es ist bekannt, Körperhohlräume oder -gefäße wie Arterien, Venen, Eileiter oder vaskuläre Fehlbildungen wie z. B. vaskuläre Aneurysmen mittels endovaskulärer Techniken zu verschließen. Das Verschlußmittel (auch als Occlusionsmittel bezeichnet) wird dabei in der Regel mit Hilfe einer Einführhilfe durch einen Katheter in den zu verschließenden Hohlraum eingeführt und dort mittels einer von verschiedenen bekannten Techniken deponiert. Der Verschluß des Hohlraumes erfolgt schließlich durch die Bildung eines Embolus infolge des verlangsamten Blutflusses in dem durch das Verschlußmittel verkleinerten oder ausgefüllten Hohlraum.

So ist es bekannt, eine Vielzahl filamentöser Verschlußmittel, meist Wendeln oder Spiralen aus Edelstahl- oder Platinlegierungen, in vaskuläre Aneurysmen einzubringen, wo diese dann eine willkürliche Konfiguration einnehmen und das Aneurysma so verschließen. Der Nachteil dieser Verfahrensweise besteht darin, daß die Verschlußmittel den Hohlraum oft nur ungenügend ausfüllen und stabilisieren und das Verhalten der Verschlußmittel bei der Ausbildung übergeordneter Strukturen schwer vorhersagbar ist, was sich letztlich negativ auf die Operationssicherheit auswirkt, da ein Stiletteffekt, der eine Wandungsruptur nach sich ziehen kann, nicht auszuschließen ist.

Zur Umgehung dieser Nachteile ist es weiterhin bekannt, Verschlußmittel aus Formgedächtnismaterialien einzusetzen, die bei der Einbringung in den zu verschließenden Hohlraum eine definierte Sekundär- und/oder Tertiärstruktur einnehmen.

So ist es zur möglichst effektiven Ausfüllung zu verschließender Gefäßaneurysmen bei gleichzeitiger Stabilisierung der Aneurysmenwandung aus der WO 01/93937 bekannt, ein Verschlußmittel aus einem Material mit Formgedächtniseigenschaften einzusetzen, welches bei der Einbringung die Primärstruktur eines langgestreckten Filamentes aufweist, das bei Einbringung in das zu verschließende Aneurysma als Sekundärstruktur sechs gleich große Schleifen ausbildet, die zusammengenommen eine 3-dimensionale Tertiärstruktur in Form eines Käfigs oder Würfels einnehmen. Jede der Schleifen bildet dabei eine Fläche des Raumkörpers und bestimmt durch ihre Größe dessen Dimensionierung.

Dieses Prinzip ist auch aus der WO 03/017852 bekannt, bei der die Implantate nach Wegfall eines äußeren Zwanges eine regelmäßige meanderförmige Sekundärstruktur einnehmen, die wiederum eine räumliche Tertiärstruktur, etwa in Form eines geometrischen Käfigs, Kubus, Tetraeders oder Prismas, ausbildet.

Die US 2004/0045554 A1 offenbart ein Implantat mit Schleifen, die weitgehend in einer Ebene liegen. Hierdurch soll erreicht werden, dass die einzelnen Schleifen nach Einbringen in ein Aneurysma Kräfte auf die Aneurysmenwandung ausüben.

Die US 2002/0019647 A1 offenbart ein Implantat, dem eine 3D-Struktur aufgeprägt wurde. Die Struktur kann weitgehend unregelmäßig, sphärisch, oval, elliptisch, kleeblattförmig oder kastenartig sein. Die zweiteige Form des Anspruchs 1 basiert such auf diesem Stand der Technik.

Derartige Verschlussmittel sollen die Stabilisierung der Aneurysmenwandung gewährleisten, so dass nachfolgend weitere filamentöse Verschlussmittel ohne die Gefahr einer Wandungsruptur eingebracht werden können. Zwar stellen diese Verschlussmittel eine Verbesserung gegenüber nicht vorgeformten Verschlussmitteln im Sinne erhöhter Operationssicherheit dar, jedoch sind insbesondere die an die Flächen und Eckbereiche, aber auch an die Kanten der Polyeder angrenzenden Bereiche der Aneurysmenwandung insbesondere durch die anschließend in das Aneurysma eingeführten Verschlußwendeln noch stark rupturgefährdet. Des weiteren kann die weitgehend offen ausgestaltete Tertiärstruktur derartiger Verschlußmittel den Austritt der nachfolgend eingeführten Verschlußmittel oder Verschlußmittelteile aus dem Aneurysmenhals im wesentlichen nur bei Aneurysmen mit kleinen Hälsen verhindern.

Angesichts der mit dem Stand der Technik verbundenen Nachteile besteht die Aufgabe der Erfindung in der Bereitstellung eines Implantates, welches für den Patienten das Risiko beim Verschluß von Körperhohlräumen und -gefäßen weiter vermindert. Wünschenswert sind eine hohe Bedeckungsdichte der Wand des Aneurysmas, eine gute Anschmiegung an die Aneurysmawand, ein zuverlässiger Verschluß des Aneurysmahalses und/oder die Verhinderung des Stiletteffekts.

Diese Aufgabe wird erfindungsgemäß durch ein medizinisches Implantat gemäß Anspruch 1 gelöst.

Die Erfindung beruht auf Ergebnissen, die belegen, daß die Erzielung einer höheren Packungsdichte des Polyeders durch Einführung zusätzlicher Schlingen das Risiko einer Wandungsruptur vermindert, ohne daß die Manövrierbarkeit des Implantates bei der Implantation signifikant verschlechtert wird. Die zusätzlichen Schlingen sind etwas kleiner in ihrem Durchmesser als die Schlingen, die die Polyederflächen ausmachen.

Das Implantat ist dabei derartig vorgeformt, daß es nach Entlassung aus dem Katheter die gewünschte Sekundär- und Tertiärstruktur ausbildet. Hierzu wird die Wendel, zumindest jedoch der Teil, der den Polyeder ausbildet, auf im Stande der Technik bekannte Art und Weise unter eine elastische Vorspannung gesetzt. Das Implantat gibt daher erst nach Wegfall eines äußeren (thermischen oder mechanischen) Zwanges seine gestreckte Struktur auf und nimmt seine vorbestimmte dreidimensionale Tertiärstruktur ein. Der mechanische Zwang kann dabei beispielsweise vom Katheter oder einem im oder um die Wendel verlaufenden Halteelement ausgeübt werden. Der thermische Zwang kann beispielsweise in einer gegenüber der Temperatur im Blutstrom unterschiedlichen Temperierung im Katheter bestehen. Derartige Mittel und Zusammenhänge sind dem zuständigen Fachmann hinlänglich bekannt.

Mit Hilfe derartig vorgeformter erfindungsgemäßer Implantate ist es möglich, eine dichte und schonende Ausfüllung des zu verschließenden Hohlraumes zu erzielen, ohne daß die Wandung des Hohlraumes bei der Ausbildung der gewünschten 3-dimensionalen Struktur als Widerlager dienen muß, wie es bei nicht vorgeformten Implantaten der Fall ist. Das Risiko einer Wandungsruptur wird so minimiert.

Zur Ausbildung einer derartigen elastischen Vorspannung eignen sich besonders Formgedächtnismaterialien bzw. Materialien mit superelastischen Eigenschaften, die eine temperatur- oder spannungsinduzierte martensitische Transformation oder eine Kombination von beiden durchlaufen können. Auch andere Materialien ohne Formgedächtniseigenschaften, wie zum Beispiel Platinlegierungen, insbesondere Platin/lridium- und PlatinlWolfram-Legierungen sind zur Ausbildung erfindungsgemäßer Implantate geeignet.

Die weitere Schlinge oder weiteren Schlingen können dabei im Polyeder auf einer bzw. mehreren Flächen des Polyeders angeordnet sein. So kann bzw. können beispielsweise innerhalb einer durch eine größere Schlinge gebildeten Fläche des Polyeders eine oder auch mehrere kleiner dimensionierte Schlinge(n) angeordnet sein. Dies führt zu einer dichteren Packung der Polyederflächen, was das Risiko der Ruptur der angrenzenden Gefäß- bzw. Aneurysmenwandung durch zusätzlich eingebrachte filamentöse Verschlußmittel minimiert. Zudem wird der Verschluß des Aneurysmenhalses verbessert, so daß die Gefahr des Austrittes zusätzlich eingebrachter filamentöser Verschlußmittel ebenfalls gesenkt wird.

Des weiteren kann bzw. können die weitere Schlinge bzw. weiteren Schlingen auf den Kanten des Polyeders angeordnet sein, um so eine dichtere Packung der Polyederkanten mit den vorstehend genannten Vorteilen in bezug auf angrenzende Aneurysmenbereiche zu ermöglichen.

Außerdem kann bzw. können die weitere Schlinge bzw. die weiteren Schlingen auf den Eckbereichen des Polyeders angeordnet sein, um so die Packungsdichte an diesen Stellen zu erhöhen, was ebenfalls die vorgenannten Vorteile in bezug auf angrenzende Aneurysmenbereiche bringt.

Die drei vorgenannten Anordnungsmöglichkeiten der weiteren Schlingen sind bei Ausführungsformen mit mehr als einer weiteren Schlinge nicht notwendigerweise fakultativ sondern auch kumulativ anwendbar, um eine optimal an den zu okkludierenden Hohlraum angepaßte Packung des Polyeders zu erzielen. Ein Ziel dabei ist vorrangig die Verlegung des Aneurysmahalses, um ein Ausschwemmen der Spiralen zu verhindern.

Gemäß einer zweckmäßigen Ausführungsform des erfindungsgemäßen Implantates ist der Polyeder ein regulärer oder halbregulärer Polyeder. Im Falle eines halbregulären Polyeders werden auch die Flächen selbst durch unterschiedlich groß dimensionierte Schlingen gebildet. Dabei können auch innerhalb der durch kleinere Schlingen gebildeten kleineren Polyederflächen wiederum kleiner dimensionierte Schlingen zur Erzielung einer dichteren Flächenpackung angeordnet sein. Darüber hinaus können die vorgenannten Maßnahmen zur dichteren Packung der Eck- und/oder Kantenbereiche zweckmäßigerweise auch hier eingesetzt werden.

Es ist dabei besonders zweckmäßig, wenn der Polyeder ein Tetraeder, ein Hexaeder (vorzugsweise ein Kubus), ein Oktaeder, ein Dodekaeder oder ein Ikosaeder ist. Im Rahmen der vorliegenden Erfindung ist ein Tetraeder dabei besonders bevorzugt.

Die Schlingen können Schleifen oder Schlaufen sein. Bei einer Schleife überkreuzen sich das proximale und distale Ende des Teils des Filamentes, das die Schleife ausbildet. Bei der Ausbildung einer Schlaufe findet diese Überkreuzung nicht statt. Aufgrund der erhöhten Stabilität der durch die Schlingen gebildeten Tertiärstruktur, ist es vorteilhaft, wenn zumindest eine der Schlingen und vorzugsweise alle Schlingen Schleifen sind.

Gemäß einer weiteren bevorzugten Ausführungsform beträgt das Größenverhältnis der kleinen und großen Schlingen 1:1,1 bis 1:5, vorzugsweise 1:1,1 bis 1:4 und besonders bevorzugt 1:1,1 bis 1:2. Die Dimensionierung hängt dabei unter anderem von der Anordnung der Schlingen an den Flächen/Kanten oder Ecken ab. Das Implantat kann dabei aus Schlingen zweier oder mehrerer unterschiedlicher Größen bestehen. In der Regel sind bei Anordnung von zwei Schlingen auf einer Fläche die Größenverhältnisse im Bereich von 1:1,1 bis 1:2. Bezugsgröße ist der Durchmesser.

Gemäß einer weiteren bevorzugten Ausführungsform weist das Implantat mehr als eine kleinere Schlinge auf. Besonders bevorzugt im Sinne einer die Operationssicherheit erhöhenden Packungsdichte ist ein Zahlenverhältnis kleinerer zu größerer Schlingen von mindestens 1:1.

Bei einem Zahlenverhältnis kleiner zu großen Schlingen von 1:1 ist es dabei bevorzugt, wenn kleine und große Schlingen in linearer Erstreckung des Filamentes abwechselnd angeordnet sind. Diese Anordnung stellt eine weitere Erhöhung der Behandlungssicherheit dar, weil sie besonders gut manövrierbar ist und überraschenderweise ein teilweises Zurückziehen des implantierten Filamentes aus dem Hohlraum in den Katheter zur Repositionierung während des Implantationsvorganges ermöglicht, ohne daß es zur Bildung von Verkantungen, Knoten oder Brüchen im Filament kommt.

Gemäß einer besonders bevorzugten Ausführungsform ist im Polyeder in jeder der durch die großen Schlingen gebildeten Polyederflächen eine kleinere Schleife angeordnet.

Des weiteren ist es besonders vorteilhaft, wenn im Polyeder zwischen jeweils zwei aneinandergrenzenden, die Flächen bildenden Schlingen jeweils mindestens eine (es können auch mehrere sein) kleinere Schlinge angeordnet ist. Die zwischen jeweils zwei aneinandergrenzenden, die Flächen bildenden Schlingen angeordneten Schlingen kommen dabei auf den Kanten des Polyeders zu liegen.

Darüber hinaus ist es besonders vorteilhaft, wenn im Polyeder an allen Bereichen, an denen jeweils mindestens drei die Flächen bildende Schlingen aneinandergrenzen, jeweils mindestens eine kleine Schlinge (es können auch mehr sein) angeordnet ist. Die zwischen jeweils mindestens drei aneinandergrenzenden, die Flächen bildenden Schlingen angeordneten Schlingen kommen dabei auf den Ecken des Polyeders zu liegen.

Es ist weiterhin besonders zweckmäßig, wenn der Polyeder ein Tetraeder ist, dessen Flächen durch je eine der großen Schlingen gebildet werden, wobei weiterhin in jeder großen Schlinge eine kleinere Schlinge angeordnet ist.

Gemäß einer weiteren bevorzugten Ausführungsform ist der Polyeder ein Tetraeder, dessen Flächen durch je eine der großen Schlingen gebildet werden, wobei zwischen jeweils zwei großen Schlingen eine kleinere Schlinge an jeweils einer Kante des Tetraeders angeordnet ist.

Eine weitere bevorzugte Ausführungsform betrifft ein erfindungsgemäßes medizinisches Implantat, bei dem der Polyeder ein Tetraeder ist, dessen Flächen durch je eine der großen Schlingen gebildet werden, wobei zwischen jeweils drei großen Schlingen eine kleinere Schlinge an einer Ecke des Tetraeders angeordnet ist.

Zum Verschluß von Aneurysmen ist es besonders zweckmäßig, erfindungsgemäße Implantate einzusetzen, deren Polyeder einen Durchmesser von 0,5 bis 30, vorzugsweise 1 bis 25 und besonders bevorzugt 2 bis 20 und insbesondere 3 bis 18 mm aufweist.

Des weiteren ist es vorteilhaft, wenn der Polyeder größer dimensioniert ist als das Volumen des Körperhohlraumes (der sogenannte "therapeutische Raum"), für dessen Ausfüllung er bestimmt ist. Dieses sogenannte "Oversizing" dient der Stabilisierung des Implantates in der zu verschließenden Cavität und sichert so gegen ein Verrutschen innerhalb derselben oder gegen einen teilweisen oder vollständigen Austritt aus dieser. Um eine Ruptur der dünnwandigen Aneurysmenwandung zu vermeiden, ist es jedoch zweckmäßig, den Polyeder in bezug auf den jeweiligen therapeutischen Raum nicht zu groß zu dimensionieren. Demgemäß ist es vorteilhaft, wenn der Durchmesser des Polyeders nicht mehr als 2,5-fach, vorzugsweise 1,1 bis 2-fach und besonders bevorzugt 1,2 bis 1,5-fach größer dimensioniert ist als der Durchmesser des Körperhohlraumes, für dessen Ausfüllung er bestimmt ist.

Das erfindungsgemäße Implantat eignet sich demgemäß insbesondere zum Verschluß von Aneurysmen mit einem therapeutischen Maß (also einem Durchmesser) von 0,4 bis 40, vorzugsweise von 1,5 bis 20 und insbesondere von 2 bis 18 mm.

Gemäß einer zweckmäßigen Ausführungsform weist das Filament (wenn nur ein Teil des Filamentes den Polyeder ausbildet, dann insbesondere dieser Teil) im ausgestreckten Zustand eine Länge von 50 bis 600 und vorzugsweise von 75 bis 500 mm auf.

Das erfindungsgemäße Implantat kann dabei beispielsweise durch eine durch mindestens einen Draht gebildete Wendel oder Spirale oder eine aus mindestens zwei Drähten gebildete kabelartige Struktur gebildet werden. Ausgestaltungen als Wendel oder Feder oder kabelartige Struktur bieten dabei den Vorteil, eine vergrößerte Oberfläche für die Thrombosierung bereitzustellen. Zum gleichen Zweck können auch weitere Ausgestaltungen der Wendel eingesetzt werden, die der Oberflächenvergrößerung dienen, beispielsweise die Ausrüstung mit die Thrombusbildung fördernden Fasern.

Der oder die Drähte weisen dabei zweckmäßigerweise einen Durchmesser von 20 bis 200, vorzugsweise 30 bis 100, besonders bevorzugt 50 bis 70 und insbesondere 55 bis 65 µm auf.

Gemäß einer zweckmäßigen Ausführungsform ist die Wendel oder Spirale mit einem inneren Lumen ausgebildet, welches zumindest zum distalen Ende hin geschlossen ist. Das Lumen kann dabei nach proximal offen oder geschlossen sein. Ein nach proximal offenes Lumen ist beispielsweise zweckmäßig, wenn im inneren Lumen des Filamentes ein entfernbares Halteelement angeordnet ist, welches die Einnahme der vorgeprägten Sekundär- und Tertiärstruktur unterbindet, solange es sich im Implantat befindet. Ein derartiges Halteelement ist offenbart in der Schrift WO 03/041615, auf deren Offenbarungsgehalt hier ausdrücklich Bezug genommen wird.

Es ist weiterhin zweckmäßig, wenn die Wendel einen Außendurchmesser von 0,1 bis 0,5, vorzugsweise 0,2 bis 0,35 und besonders bevorzugt 0,24 bis 0,28 mm aufweist. Hierbei ist die Wendel zweckmäßigerweise als Mikrowendel bzw. Mikrofeder aus einem oder mehreren Drähten ausgebildet oder als kabelartige Struktur aus mehr als einem Draht verflochten oder verdrillt.

Gemäß einer weiteren zweckmäßigen Ausführungsform ist mindestens einer der die Wendel bildenden Drähte bzw. der Draht aus einer Platinlegierung, vorzugsweise einer Platin/Iridium- oder einer Platin/Wolfram-Legierung oder einer metallischen Legierung mit Formgedächtniseigenschaften ausgebildet.

Es kann zweckmäßig sein, wenn die Wendel in Längsachse von einem filamentösen Formelement aus einer metallischen Legierung mit Formgedächtniseigenschaften durchzogen ist. Das Formelement dient dabei der Ausbildung der Sekundär- und Tertiärstruktur des Implantates nach Verlassen des Katheters. Bei dieser Ausführungsform paßt sich das Filament nach Entlassung aus dem Katheter der aufgegebenen Form des Formelementes an, so daß es zur Ausbildung der vorgegebenen 3-dimensionalen Tertiärstruktur kommt. Ein derartiges Formelement ist beispielsweise offenbart in der Schrift WO 03/017852, auf deren Offenbarungsgehalt hier explizit Bezug genommen wird.

Bei dieser Ausführungsform ist die Wendel zweckmäßigerweise als Kabel aus mehreren Drähten, von denen einer das Formelement ist, und besonders bevorzugt als Spirale oder Wendel ausgebildet, in deren innerem Lumen das Formelement, vorzugsweise ein Draht, verläuft. Bei dieser Ausführungsform ist es weiterhin zweckmäßig, wenn die Spirale bzw. Wendel, bzw. bei einer Ausführung als Kabel die Teile des Kabels, die nicht das Formelement darstellen, aus einem Material ohne Formgedächtniseigenschaften bestehen. Besonders zweckmäßig ist hier eine Platin/Iridium-Legierung.

Die Legierung mit Formgedächtniseigenschaften ist dabei vorzugsweise eine Titan und Nickel enthaltende Legierung (auch bekannt als Nitinol), eine Eisenbasis- oder Kupferbasislegierung.

Gemäß einer weiteren bevorzugten Ausführungsform weist das filamentöse Formelement, das sich entlang der Längsachse der Wendel erstreckt, eine Verjüngung in seinem distalen Endbereich auf. Das Formelement hat somit hier einen geringeren Durchmesser, als im weiteren Verlauf nach proximal. Eine solche distale Taperung bewirkt, daß das Formelement weicher und weniger steif wird, wodurch sich die Gefahr einer Traumatisierung bei Kontakt des distales Endes des Formelements mit der Aneurysmawand verringert. Der distalen Taperung liegt die Erkenntnis zugrunde, daß die Gesamtsteifigkeit einer Wendel, die von einem filamentösen Formelement durchzogen ist, zum größeren Teil durch das Formelement und nur in geringerem Maße durch die Wendel selbst bestimmt wird. Die Verjüngung kann sich z. B. über einen Bereich von ca. 20 mm, von der distalen Spitze an gerechnet, erstrecken und dabei stufenweise, vorzugsweise aber kontinuierlich erfolgen. Insgesamt nimmt der Durchmesser des Formelements bis zu seiner distalen Spitze vorzugsweise auf ca. 25 bis 50 % des Durchmessers im sonstigen Bereich ab.

Eine weitere zweckmäßige Ausführungsform betrifft ein erfindungsgemäßes medizinisches Implantat, bei dem die Wendel in Längsachse von einem filamentösen Halteelement aus einem Polymer (insbesondere einem Polyester oder Polyamid) oder einem metallischen Draht ohne Formgedächtniseigenschaften (insbesondere einem medizinischen Edelstahldraht) durchzogen ist. Das Halteelement dient in diesem Fall zum Festhalten in gestreckter Konfiguration (in diesem Fall ist eine Ausbildung des Halteelementes als metallischer Draht zweckmäßig, insbesondere in Kombination mit einer Wendel aus einem Formgedächtnismaterial) oder zur Sicherung der Wendel gegen ein Abreißen bei der Implantation, insbesondere bei der Repositionierung. Im letzteren Falle ist eine Ausbildung des Halteelementes aus einem Polymer und der Wendel aus einem Formgedächtnismaterial oder aus einer Platin/Wolfram-Legierung besonders zweckmäßig. Aufgrund seines guten Abstützungsverhaltens im Sinne einer verbesserten Schiebbarkeit des Implantates ist die Ausbildung der Wendel aus einer Platin/Wolfram-Legierung dabei besonders bevorzugt.

Die vorliegende Erfindung betrifft weiterhin eine Vorrichtung zur Implantation von Implantaten in Körpergefäßen und Hohlräumen mit einem erfindungsgemäßen Implantat und einer Einführhilfe, welche lösbar mit dem proximalen Ende des Implantates verbunden ist.

Die Einführhilfe ist dabei vorzugsweise als Führungsdraht ausgebildet. Dieser ist zweckmäßigerweise zumindest teilweise als Wendel oder Feder ausgebildet Die Dimensionierung und Auswahl geeigneter Materialien ist dem zuständigen Fachmann hinlänglich bekannt. Beispielhaft wird hierzu explizit auf den Offenbarungsgehalt der Schriften WO 03/017852 und WO 03/041615 verwiesen.

Weist das erfindungsgemäße Implantat ein herausnehmbares Halteelement auf, ist sie zweckmäßigerweise als offene Röhre ausgebildet, durch die das Halteelement in und aus dem Implantat manövriert werden kann.

Zur Deponierung des Implantates im zu verschließenden Hohlraum sind Implantat und Einführhilfe zweckmäßigerweise durch ein Ablösemodul miteinander verbunden. Für die elektrolytische Deponierung des Implantates ist das Ablösemodul zweckmäßigerweise mit einer elektrolytisch korrodierbaren Stelle aus einem geeigneten Material, z. B. einer korrodierbaren Stahllegierung, versehen.

Darüber hinaus können im erfindungsgemäßen Implantat noch ein oder mehrere Ablösemodule angeordnet sein, die in der Wendel proximal zum den Polyeder ausbildenden Teil angeordnet ist bzw. sind. Dies ermöglicht die Verfüllung des inneren Hohlraumes des Polyeders im direkten Anschluss an dessen Einbringung in den zu verschließenden Körperhohlraum. Der zwischen Polyeder und Ablösemodul angeordnete Filamentabschnitt kann dabei seinerseits elastisch vorgeformt sein, um im Polyederhohlraum eine definierte Form bzw. Position einzunehmen. Die Anordnung mehrerer derartiger Ablösemodule in der Wendel ermöglicht die Einbringung variabel dimensionierbarer Wendelabschnitte in das Polyederlumen. Für die elektrolytische Deponierung des Implantates ist das Ablösemodul zweckmäßigerweise mit einer elektrolytisch korrodierbaren Stelle versehen.

Es ist weiterhin zweckmäßig, wenn die erfindungsgemäße Vorrichtung weiterhin einen Katheter, eine Spannungsquelle und eine Kathode aufweist, wobei das Implantat als Anode dient und im Katheter in Längsrichtung verschiebbar ist und wobei die Verbindung von Implantat und Einführhilfe (vorzugsweise das Ablösemodul) eine elektrolytisch korrodierbare Stelle aufweist, so daß das Implantat im Kontakt mit einer Körperflüssigkeit durch elektrolytische Prozesse abtrennbar ist.

Das Ablösemodul weist dabei zur Gewährleistung einer hohen Bruchsicherheit bei gleichzeitig guter Ablösbarkeit einen Durchmesser von 30 bis 150, vorzugsweise 40 bis 120 und besonders bevorzugt 50 bis 100 µm auf. Die elektrolytisch korrodierbare Stelle kann dabei einen geringeren Durchmesser als die angrenzenden proximalen und distalen Bereiche des Ablösemoduls aufweisen (das Ablösemodul verjüngt sich in diesem Fall zur elektrolytisch korrodierbaren Stelle hin).

Das Ablösemodul umfaßt zweckmäßigerweise eine proximale und eine distale Wendel sowie ein dazwischenliegendes Segment, wobei die Wendeln aus einem Material bestehen, welches gegenüber der elektrolytischen Korrosion weniger anfällig ist als das des zwischenliegenden Segmentes. Geeignete Materialkombinationen sind zweckmäßigerweise Edelmetalle oder Edelmetall-Legierungen, vorzugsweise Platin oder Platin-Legierungen für die proximale bzw. distale Wendel und rostfreier Stahl (z. B. die Typen AISI 301, 303 oder 316 sowie deren Untergruppen oder N-legierte austenitische Stähle in nicht rostender Qualität, vorzugsweise aus der Gruppe druckaufgestickter Stähle) für das zwischenliegende Segment. Derartige Materialkombinationen sind ebenfalls aus der Schrift WO 03/017852 bekannt, auf die hier Bezug genommen wird.

Gemäß einer vorteilhaften Ausführungsform der erfindungsgemäßen Vorrichtung ist das Ablösemodul mit dem Implantat bzw. der Einführhilfe durch Verschweißen, Verlöten, Verkleben oder mechanisches, insbesondere kraft- bzw. formschlüssiges Fügen auf dem Fachmann bekannte Weise unlösbar verbunden.

Gemäß einer weiteren vorteilhaften Ausgestaltung der erfindungsgemäßen Vorrichtung ist die Einführhilfe zumindest teilweise von einem elektrisch isolierenden Schrumpfschlauch oder einer elektrisch isolierenden Beschichtung umgeben.

Die erfindungsgemäßen Zwecke können weiterhin besonders vorteilhaft mit einer Wendel erzielt werden, deren Schlingen die Flächen eines Dodekaeders, insbesondere eines regelmäßigen Pentagondodekaeders ausbilden. Aufgrund der Vielzahl der Flächen - insgesamt zwölf - wird eine sehr dichte Belegung der Wand erzielt, so daß auch gegebenenfalls ohne zusätzliche Schlingen eine hohe Deckung der Aneurysmawand und eine weitgehende Verlegung des Aneurysmahalses erzielt werden können.

Die in Form eines Pentagondodekaeders ausgebildete und vorgeformte Wendel bildet dabei ein die Wand abdeckendes und schützendes flexibles Gerüst, das geeignet ist, weitere zur Okklusion bestimmte Wendeln in sich aufzunehmen und festzuhalten. Eine zusätzliche Belegung mit weiteren Schlingen auf den Flächen, Kanten oder Ecken ist häufig nicht mehr erforderlich. Insoweit kann auf gleitende Maßnahmen, die einer Traumatisierung des Patienten entgegen wirken sollen, weitgehend verzichtet werden.

Als besonders vorteilhaft hat es sich herausgestellt, das proximale Ende der den Pentagondodekaeder bildenden Wendel in einem Eckpunkt vorzusehen. Da der Aneurysmahals in der Regel innerhalb einer Fläche des Polyeders liegt, wird hierdurch ein "Herausschlüpfen" des zuletzt eingebrachten Teils der Wendel aus dem Hals und ein Ausschwemmen der Wendel erschwert, die ohnehin aufgrund der Vielzahl der Flächen, der engen Anlehnung an die Aneurysmawand und der in der Regel herrschenden Verspannung im Inneren des Aneurysmas unwahrscheinlich ist. Das distale Ende der Wendel liegt dabei vorzugsweise in einer Fläche und bildet den Anfang der ersten Schlinge; hier vorteilhaft eine im wesentlichen atraumatische Lagerung auf einer vom Aneurysmahals abgewandten Seite des Aneurysmas.

Die Erfindung wird nachfolgend beispielhaft anhand der in den Zeichnungen veranschaulichten Ausführungsbeispiele näher erläutert. Es stellen dar:
- Figur 1: die vergrößerte Darstellung eines kubusförmigen Implantates 1 des Standes der Technik;
- Figur 2a und 2b: die Darstellung eines erfindungsgemäßen Implantates 1' mit kleineren Schleifen 8 in den Tetraederflächen 2' in vergrößerter Darstellung;
- Figur 3: schematische Darstellung eines erfindungsgemäßen Implantates 1' mit kleineren Schleifen 8 in den Tetraederflächen 2' als Abwicklung einer Kugel in vergrößerter Darstellung;
- Figur 4a und 4b: die Darstellung eines erfindungsgemäßen Implantates 1' mit kleineren Schleifen 8 an den Ecken 4' eines Tetraeders 6 in vergrößerter Darstellung;
- Figur 5: die schematische Darstellung eines erfindungsgemäßen Implantates 1' mit kleineren Schleifen 8 an den Ecken 4' eines Tetraeders 6 als Abwicklung einer Kugel in vergrößerter Darstellung;
- Figur 6: die Vertikalansicht eines in ein Beerenaneurysma 13 implantierten erfindungsgemäßen Implantates 1' in vergrößerter Darstellung;
- Figur 7: die schematische Darstellung einer Abwicklung eines erfindungsgemäßen Implantates in Form eines Pentagondodekaeders; und
- Figur 8: die Verjüngung im distalen Endbereich eines filamentösen Formelements.

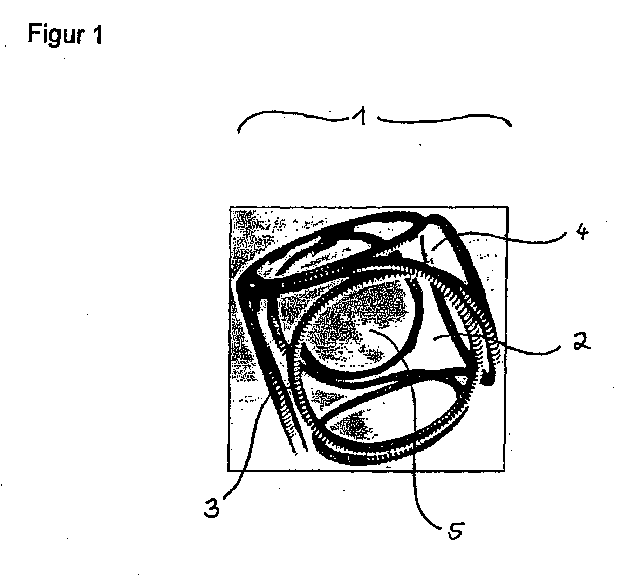

Die Figur 1 stellt ein kubusförmiges Implantat 1 des Standes der Technik in vergrößertem Format dar. Die offene Ausgestaltung insbesondere der Flächen 2 und Eckbereiche 4, aber auch der Kanten 3 stellt eine Schwachstelle derartiger Implantate 1 dar, da die hieran angrenzende Aneurysmenwandung besonders rupturgefährdet ist. Darüber hinaus verhindert die ungenügende Packungsdichte des Implantates 1 an den genannten Bereichen 2, 3 und 4 einen Wiederaustritt von zur Ausfüllung des inneren Hohlraumes 5 nachgeschobener Implantate aus dem Aneurysmenhals nur in geringem Maße.

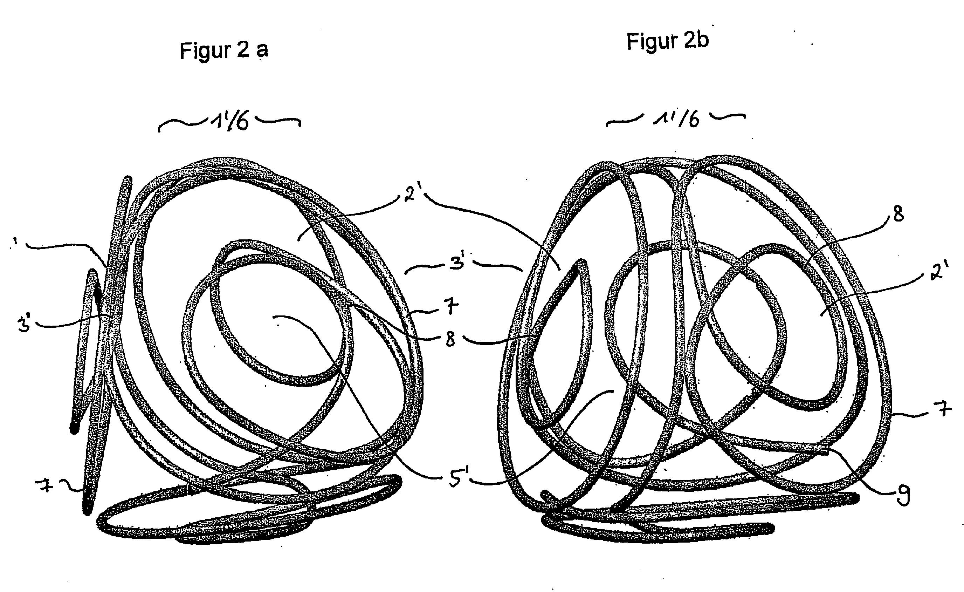

Die Figur 2 stellt in zwei Ansichten 2a und 2b ein tetraederförmiges erfindungsgemäßes Implantat 1' nach Einnahme seiner 3-dimensionalen tetraedrischen Tertiärstruktur dar. Die Flächen 2' des Tetraeders 6 werden durch gleichförmig dimensionierte große Schleifen 7 gebildet, von denen jeweils zwei aneinander grenzen, wobei die Projektionen der großen Schleifen 7 in den Raum an den Schnittflächen jeweils zweier benachbarter großer Schleifen 7 die gedachten Kanten 3' des Tetraeders 6 bilden.

In jeder der Flächen 2' ist eine kleiner dimensionierte Schleife 8 angeordnet. Diese Anordnung erhöht die Packungsdichte des Tetraeders 6 im Bereich der Flächen 2', wodurch die angrenzende Aneurysmenwandung verstärkt gegen eine Ruptierung durch nachfolgend eingeschobene Implantatteile oder weitere Implantate geschützt wird. Darüber hinaus werden durch die so erreichte hohe Packungsdichte der Flächen 2' insbesondere nachfolgend eingeschobene Implantatteile bzw. nachfolgend eingeschobene weitere Implantate zur Ausfüllung des inneren Hohlraumes 5' gegen einen Wiederaustritt aus dem Aneurysmenhals gesichert. Das erfindungsgemäße Implantat 1' eignet sich demgemäß insbesondere auch zum therapeutischen Verschluß von Aneurysmen mit großen Hälsen, deren Behandlung sich gewöhnlich besonders schwierig gestaltet.

Überdies ermöglicht die leicht erhabene Anordnung der kleineren Schleifen 8 über der Projektionsebene der durch die großen Schleifen 7 gebildeten Tetraederflächen2' eine besonders gute Fixierung des Implantates 1' im Aneurysma, siehe hierzu insbesondere Figur 2a.

Das den Tetraeder 6 ausbildende Filament 9 ist eine Mikrowendel von 0,26 mm Durchmesser, die aus einem Platin-Iridium-Draht von 60 µm Durchmesser besteht. Im inneren Hohlraum der Mikrowendel verläuft ein Nitinol Draht, der am proximalen und distalen Ende mit dem Filament 9 unlösbar verbunden ist und die Wendel 9 durch ihre elastische Vorspannung nach der Entlassung aus dem Katheter die tetraederförmige Tertiärstruktur aufprägt.

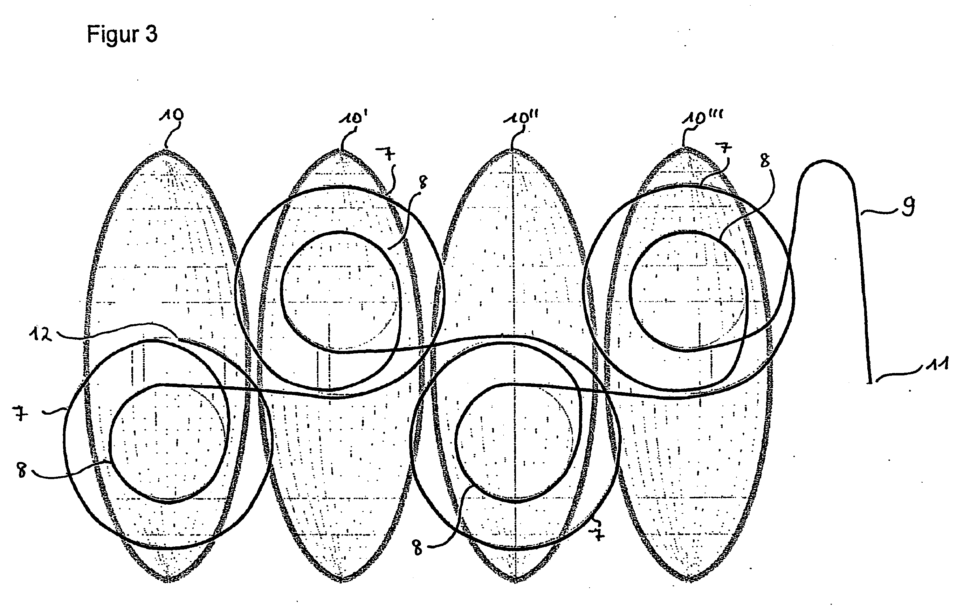

Figur 3 stellt die Sekundärstruktur des in Figur 2 dargestellten Tetraeders als Abwicklung einer Kugel mit Hilfe von 4 Radialschnitten 10 bis 10"' dar. Die Schleifen 7/8 selbst sind in etwa kreisförmig ausgebildet und formen bei ihrer vorbestimmten Anordnung im Raum einen regelmäßigen Tetraeder. Entlang der Längsachse der Wendel9 sind die großen 7 und die kleinen 8 Schleifen abwechselnd angeordnet, wobei die kleinen Schleifen 8 in der Sekundärstruktur im Inneren der großen Schleifen 7 zu liegen kommen. Mit 11 ist das proximale und mit 12 das distale Ende des Filamentes 9 bezeichnet.

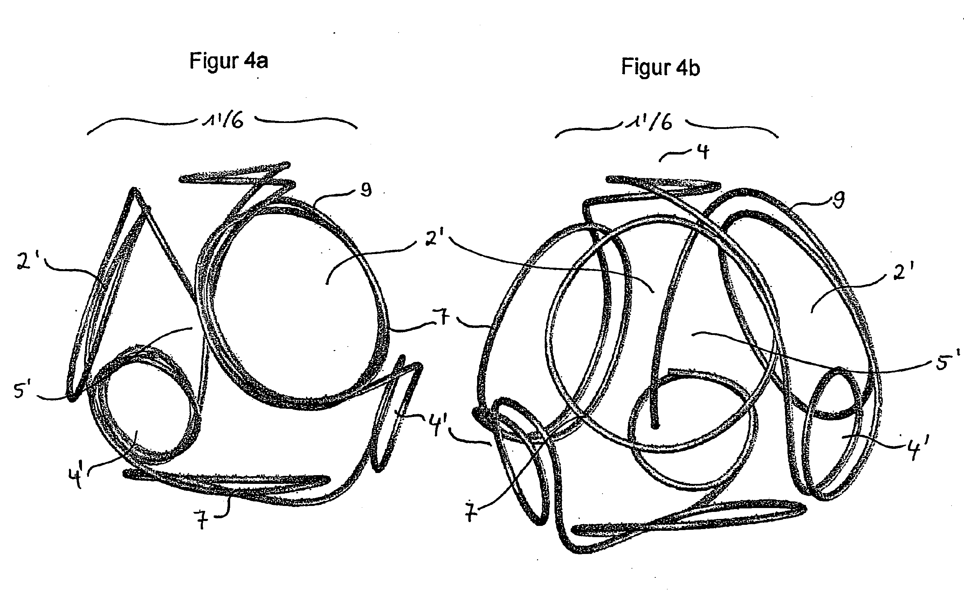

Die Figur 4 stellt in zwei Ansichten 4a und 4b ein tetraederförmiges erfindungsgemäßes Implantat 1' nach Einnahme der 3-dimensionalen Tertiärstruktur dar. Die Flächen 2' des Tetraeders 6 werden durch gleichförmig dimensionierte große Schleifen 7 gebildet, von denen jeweils zwei aneinandergrenzen und so in ihrer Projektion die gedachten Kanten 3' des Tetraeders 6 bilden. In den Schnittpunkten der Projektion jeweils dreier aneinandergrenzender großer Schleifen 7 befinden sich die Ecken 4' des Tetraeders, an denen jeweils eine kleiner dimensionierte Schleife 8 angeordnet ist. Da die Anordnung der kleineren Schleifen 7 unterhalb der gedachten Schnittpunkte liegt, weist der Tetraeder6 eine gegenüber einer idealen geometrischen Tetraederform abgerundete Form auf. Diese Anordnung erhöht einerseits die Packungsdichte des Tetraeders 6 im Bereich der Ecken 4', wodurch die daran angrenzende Aneurysmenwandung verstärkt gegen eine Ruptierung durch nachfolgend eingeschobene Implantatteile oder weitere Implantate geschützt wird. Andererseits paßt sich die so gebildete, abgerundete tetraedrische Form gefälliger in die organische Struktur auszufüllender Aneurysmenlumen ein als dies ein idealer geometrischer Tetraeder könnte. Darüber hinaus werden durch die so erreichte hohe Packungsdichte der Ecken 4' insbesondere nachfolgend zur Ausfüllung des inneren Hohlraumes 5' eingeschobene Implantatteile bzw. weitere Implantate gegen einen Wiederaustritt aus dem Aneurysmenhals gesichert.

Die den Tetraeder 6 ausbildende Wendel 9 ist eine Mikrowendel von 0,26 mm Durchmesser, die aus einem Platin-Iridium-Draht von 60 µm Durchmesser besteht. Im inneren Hohlraum der Mikrowendel verläuft ein Polymerfaden oder auch ein Faden aus einer Nickel-Titan-Legierung, der am proximalen und distalen Ende mit der Wendel 9 fixiert ist und die Wendel 9 gegen ein Abreißen während der Implantation etwa beim Repositionieren sichert. Der Platin-Iridium-Draht steht unter elastischer Vorspannung, die ihm nach Wegfall des durch den Katheter ausgeübten mechanischen Zwanges die vorgeprägte tetraedrische Gestalt aufzwingt. Die verwendete Platin-Iridium-Legierung hat zwar keine Formgedächtniseigenschaften, gewährleistet aufgrund ihres starken Abstützungsverhaltens jedoch eine hervorragende Schiebbarkeit der Wendel während der Implantation.

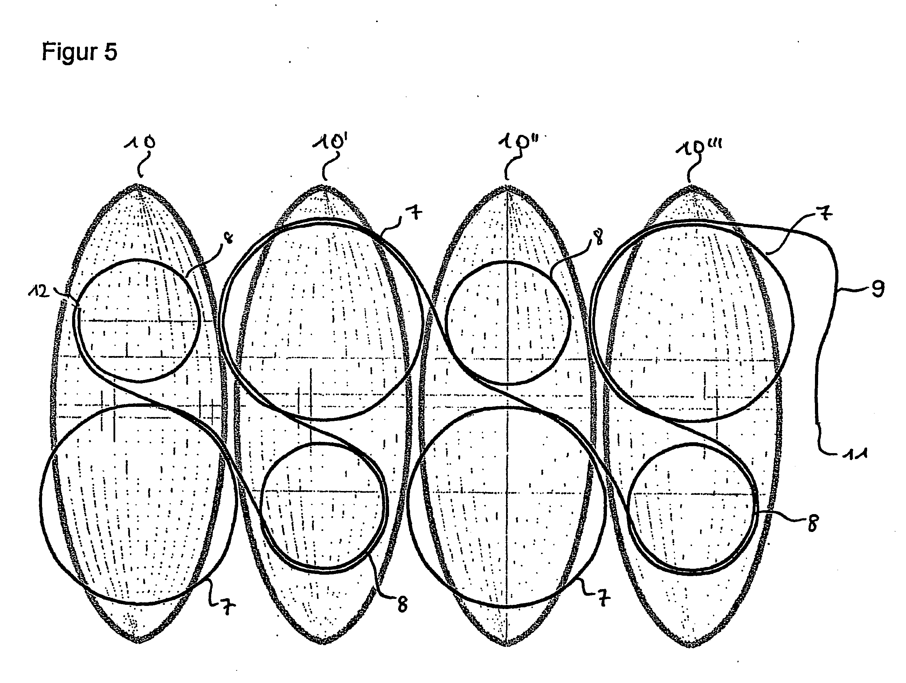

Figur 5 stellt mit Hilfe von 4 Radialschnitten 10 bis 10"' die Sekundärstruktur des in Figur 4 dargestellten Tetraeders als Abwicklung einer Kugel dar. Die Schleifen 7/8 selbst sind in etwa kreisförmig ausgebildet und formen bei der ihnen vorbestimmten Anordnung im Raum einen regelmäßigen Tetraeder. Entlang der Längsachse der Wendel 9 sind die großen 7 und die kleinen 8 Schleifen abwechselnd angeordnet, wobei die kleinen Schleifen 8 zwischen den großen Schleifen 7 zu liegen kommen. Mit 11 ist das proximale und mit 12 das distale Ende der Wendel 9 bezeichnet.

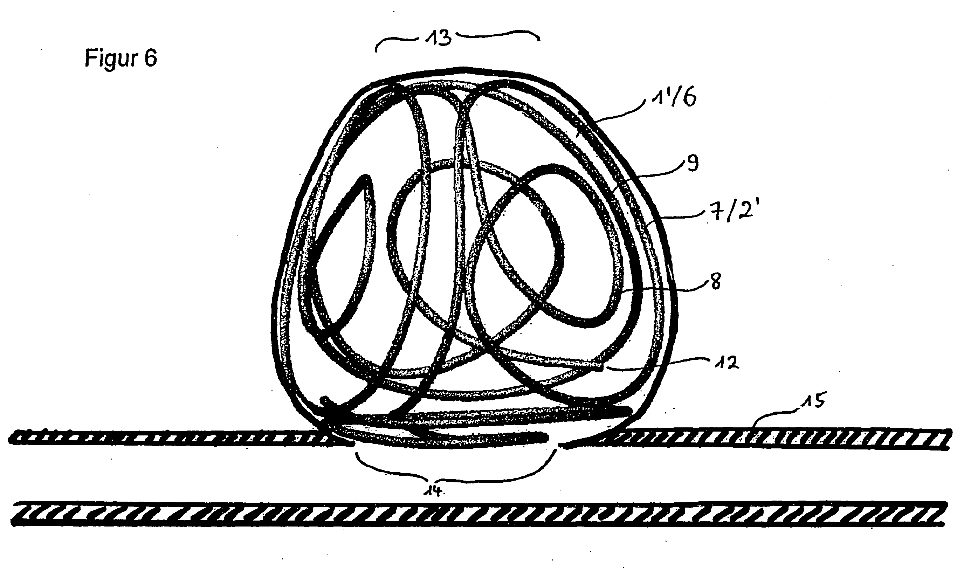

Figur 6 stellt ein in ein Beerenaneurysma 13 implantiertes erfindungsgemäßes Implantat 1' dar, welches als Tertiärstruktur einen Tetraeder 6 ausbildet. Durch die Anordnung der kleineren Schleifen 8 im Bereich der durch die großen Schleifen 7 gebildeten Flächen 2' des Tetraeders 6 wird hier eine erhöhte Packungsdichte der Tetraederflächen 2' erzielt. Dies vermindert nicht nur das Risiko einer Wandungsruptur, darüber hinaus schützt es insbesondere vor dem Wiederaustritt zusätzlich eingeschobener Implantate (nicht dargestellt) aus dem Aneurysmenhals 14. Durch diese Ausgestaltung können selbst Aneurysmen mit, wie vorliegend dargestellt, mittelgroßen Hälsen 14 auch ohne Einsatz von Stents verschlossen werden. Dazu ist es besonders zweckmäßig, wenn das Implantat 1' wie vorliegend so positioniert ist, daß eine der packungsdichten Flächenbereiche 2' des Tetraeders 6 am bzw. über den Aneurysmenhals 14 zu liegen kommt.

Die tetraedrische Tertiärstruktur eignet sich in besonderem Maße für den Verschluß großer Aneurysmen, wie etwa hier dargestellt, einem Aneurysma 13 mit einem therapeutischen Maß von 10 mm Durchmesser. Da der Tetraeder 6 einen Durchmesser von 12 mm aufweist, verspannt er sich während der Implantation bei der Ausbildung seiner Tertiärstruktur im Aneurysma 13, wobei die so aufgebaute Spannung ihn gegen ein Herausrutschen aus dem Aneurysma 13 sichert. Dieses "Oversizing" ist besonders für die Behandlung von Aneurysmen mit großen Aneurysmenhälsen vorteilhaft, weil herkömmliche Implantate darin nicht genügend gegen einen Wiederaustritt gesichert sind.

Das Implantat 1' wurde mit dem distalen Anteil 12 der Wendel 9 voran mittels eines Mikrokatheters durch das Blutgefäß 15 in das Aneurysma 13 geschoben, wo es nach Entlassung aus dem Katheter aufgrund einer gemischten spannungs- und temperaturinduzierten martensitischen Transformation des in der aus einer Platin-Iridium-Legierung bestehenden Mikrowendel 9 untergebrachten Nitinoldrahtes die dargestellte 3-dimensionale Tertiärstruktur einnahm. Nach röntgenologischer Kontrolle der korrekten Positionierung durch herkömmliche Verfahren des Standes der Technik erfolgte die elektrolytische Ablösung des Implantates von der als Führungsdraht ausgebildeten Einführhilfe. Dazu wurde mit Hilfe einer Spannungsquelle, einer auf der Körperoberfläche positionierten Kathode und dem als Anode dienenden, im zu verschließenden Aneurysma 13 positionierten Implantat 1' über einen Zeitraum von 0,1 bis 20 Minuten eine Spannung angelegt. Durch diese Spannung kam es zu einer elektrolytischen Abtrennung des Implantats 1' durch elektrolytische Korrosion an der elektrolytisch korrodierbaren Stelle des zwischen Führungsdraht und Filament 9 angeordneten Ablösemoduls. Dieses ist zur Gewährleistung einer hohen Sicherheit zur Verhinderung eines Knickens oder Ausbeulens während der Positionierung des Implantates 1 mit einem relativ großen Durchmesser von 100 µm besonders robust ausgelegt. Der Führungsdraht wurde dann zurück in den Katheter gezogen und, ebenso wie der Katheter, aus dem System entfernt.

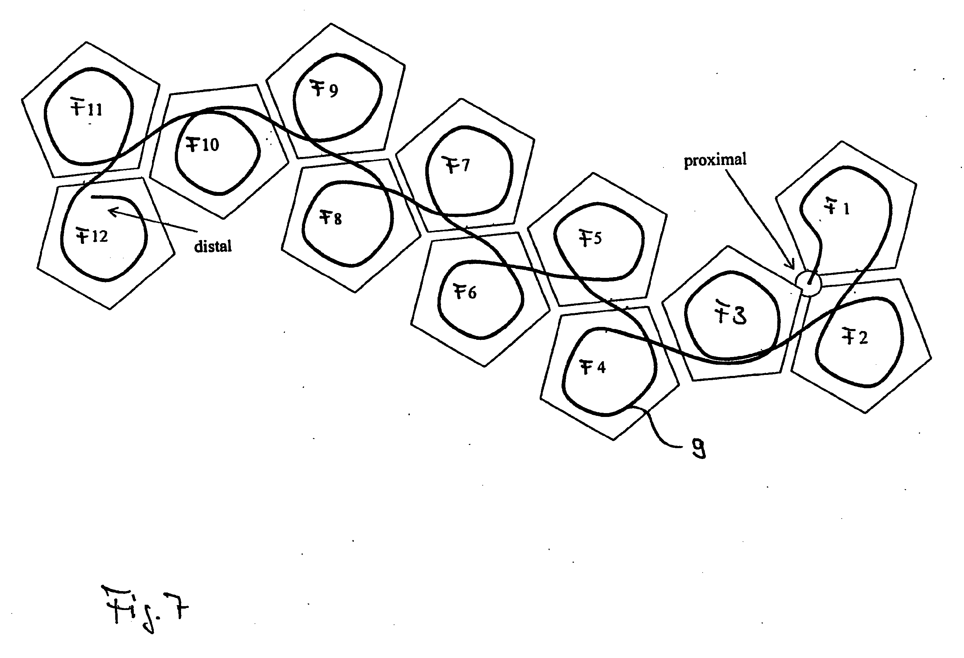

Figur 7 zeigt schematisch die Abwicklung eines Pentagondodekaeders und den Verlauf einer Mikrowendel 9 unter Ausbildung eines Pentagondodekaeders. Die einzelnen Flächen F1 bis F12 des Polyeders werden durch die Schlingen der Mikrowendel definiert. Dabei befindet sich das distale Ende der Mikrowendel 9 auf einer Fläche F12 während das proximale Ende in einem Eckpunkt zwischen F1/F2/F3 in den Körper eintaucht.

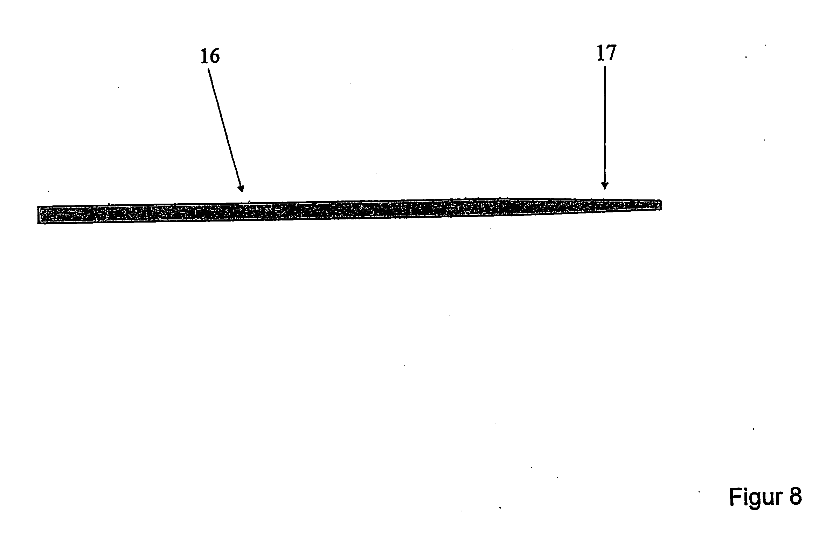

Figur 8 zeigt schließlich schematisch die Verjüngung des distalen Endes 17 eines filamentösen Formelements 16 auf ca. 50 % des Durchmessers.

## Patentansprüche

1. Medizinisches Implantat in Form einer langgestreckten Wendel (9), wobei zumindest ein Teil der Wendel (9) zur Einnahme einer Sekundärstruktur mit gleich großen Schlingen (7) und zur Ausbildung einer polyederförmigen Tertiärstruktur vorgeformt ist, die sie am Implantationsort während der Implantation einnimmt, wobei jede Fläche (2') des Polyeders von einer großen Schlinge (7) gebildet wird, wobei das Polyeder ein oder mehrere weitere, kleinere Schlingen (8) aufweist, die kleiner sind als die großen Schlingen (7), welche die Flächen (2') des Polyeders ausbilden, **dadurch gekennzeichnet, daß** die weiteren, kleineren Schlingen (8) auf einer oder mehreren Flächen (2'), Kanten (3') und/oder Ecken (4') des Polyeders angeordnet sind.

2. Medizinisches Implantat gemäß Anspruch 1, **dadurch gekennzeichnet, daß** das Polyeder ein Tetraeder (6), ein Hexaeder, ein Oktaeder, ein Dodekaeder, insbesondere ein Pentagondodekaeder, oder ein Ikosaeder ist.

3. Medizinisches Implantat gemäß einem der vorstehenden Ansprüche, **dadurch gekennzeichnet, daß** die Schlingen (7, 8) Schleifen sind.

4. Medizinisches Implantat gemäß einem der vorstehenden Ansprüche, **dadurch gekennzeichnet, daß** das Zahlenverhältnis kleinerer (8) zu großer (7) Schlingen etwa 1:1 beträgt.

5. Medizinisches Implantat gemäß Anspruch 4, **dadurch gekennzeichnet, daß** das Zahlenverhältnis kleinerer (8) zu großer (7) Schlingen 1:1 beträgt und die Schlingen (7, 8) in linearer Erstreckung der Wendel (9) abwechselnd angeordnet sind.

6. Medizinisches Implantat gemäß Anspruch 5, **dadurch gekennzeichnet, daß** im Polyeder in jeder der durch die großen Schlingen (7) gebildeten Polyederflächen (2) eine kleinere Schlinge (8) angeordnet ist.

7. Medizinisches Implantat gemäß einem der vorstehenden Ansprüche, **dadurch gekennzeichnet, daß** im Polyeder zwischen jeweils zwei der aneinandergrenzenden großen Schlingen (7) mindestens eine kleinere Schlinge (8) angeordnet ist.

8. Medizinisches Implantat gemäß einem der vorstehenden Ansprüche, **dadurch gekennzeichnet, daß** im Polyeder zwischen jeweils drei aneinandergrenzenden großen Schlingen (7) mindestens eine kleinere Schlinge (8) angeordnet ist.

9. Medizinisches Implantat gemäß einem der vorstehenden Ansprüche, **dadurch gekennzeichnet, daß** die Wendel (9) im ausgestreckten Zustand eine Länge von 50 bis 600, vorzugsweise 100 bis 500 mm aufweist.

10. Medizinisches Implantat gemäß einem der vorstehenden Ansprüche, **gekennzeichnet durch** eine Mikrowendel (9) mit einem Lumen, welches zumindest zum distalen Ende hin geschlossen ist.

11. Medizinisches Implantat gemäß einem der vorstehenden Ansprüche, **dadurch gekennzeichnet, daß** die Wendel (9) einen Außendurchmesser von 0,1 bis 0,5 mm, vorzugsweise 0,2 bis 0,35 mm und besonders bevorzugt 0,24 bis 0,28 mm aufweist.

12. Medizinisches Implantat gemäß einem der vorstehenden Ansprüche, **dadurch gekennzeichnet, daß** mindestens einer der die Wendel (9) bildenden Drähte bzw. der die Wendel (9) bildende Draht aus einer Platin-Iridium- oder einer Platin-Wolfram-Legierung oder einer metallischen Legierung mit Formgedächtniseigenschaften besteht.

13. Medizinisches Implantat gemäß einem der vorstehenden Ansprüche, **dadurch gekennzeichnet, daß** die Wendel (9) in Längsachse von einem filamentösen Formelement (16) aus einer metallischen Legierung mit Formgedächtniseigenschaften durchzogen ist.

14. Medizinisches Implantat gemäß einem der vorstehenden Ansprüche, **dadurch gekennzeichnet, daß** die Wendel (9) in Längsachse von einem filamentösen Halteelement (16) aus einem Polymer oder einem metallischen Draht ohne Formgedächtniseigenschaften durchzogen ist.

15. Medizinisches Implantat gemäß Anspruch 13 oder 14, **dadurch gekennzeichnet, daß** der Durchmesser des filamentösen Formelements (16) sich zum distalen Ende hin verringert.

16. Medizinisches Implantat gemäß Anspruch 15, **dadurch gekennzeichnet, daß** der Durchmesser des filamentösen Formelements (16) am distalen Ende 25 bis 50 % des sonstigen Durchmessers des Formelements 16) beträgt.

17. Medizinisches Implantat gemäß Anspruch 15 oder 16, **dadurch gekennzeichnet, daß** sich das filamentöse Formelement (16) im distalen Endbereich kontinuierlich verjüngt

18. Medizinisches Implantat gemäß einem der vorstehenden Ansprüche mit mindestens einem Ablösemodul mit einer elektrolytisch korrodierbaren Stelle, welches in der Wendel (9) proximal zum das Polyeder ausbildenden Teil angeordnet ist.

19. Vorrichtung zur Implantation von Implantaten (1') in Körpergefäßen und Hohlräumen mit einem Implantat (1') gemäß einem der vorstehenden Ansprüche 1 bis 18 und einer Einführhilfe, welche lösbar mit dem proximalen Ende (11) des Implantates (1') verbunden ist.

20. Vorrichtung gemäß Anspruch 19 mit einem Katheter, einer Spannungsquelle und einer Kathode, bei der das Implantat (1') als Anode dient und im Katheter in Längsrichtung verschiebbar ist, wobei die Verbindung von Implantat (1') und Einführhilfe eine elektrolytisch korrodierbare Stelle aufweist, so daß das Implantat (1') im Kontakt mit einer Körperflüssigkeit durch elektrolytische Prozesse abtrennbar ist.

21. Vorrichtung gemäß einem der Ansprüche 19 oder 20, **dadurch gekennzeichnet, daß** die Einführhilfe als Führungsdraht ausgebildet ist.

22. Vorrichtung gemäß Anspruch 21, **dadurch gekennzeichnet, daß** der Führungsdraht zumindest teilweise als Wendel oder Feder ausgebildet ist.

23. Vorrichtung gemäß einem der Ansprüche 19 bis 22, **dadurch gekennzeichnet, daß** Implantat (1') und Einführhilfe durch ein Ablösemodul miteinander verbunden sind, welches vorzugsweise eine elektrolytisch korrodierbare Stelle aufweist.

24. Vorrichtung gemäß einem der Ansprüche 19 bis 23, **dadurch gekennzeichnet, daß** das Ablösemodul eine proximale und eine distale Wendel sowie ein dazwischenliegendes Segment umfaßt, wobei die Wendeln aus einem Material bestehen, welches gegenüber der elektrolytischen Korrosion weniger anfällig ist als das des zwischenliegenden Segmentes.

25. Vorrichtung gemäß einem der Ansprüche 19 bis 24, **dadurch gekennzeichnet, daß** das Ablösemodul mit dem Implantat (1') bzw. der Einführhilfe durch Verschweißen, Verlöten, Verkleben oder mechanisches, insbesondere kraft- bzw. formschlüssiges Fügen unlösbar verbunden ist.

26. Vorrichtung gemäß einem der Ansprüche 19 bis 25, **dadurch gekennzeichnet, daß** die Einführhilfe zumindest teilweise von einem elektrisch isolierenden Schrumpfschlauch oder einer elektrisch isolierenden Beschichtung umgeben ist.

## Claims

1. Medical implant in the form of an elongate coil (9), wherein at least a part of the coil (9) is preformed in order to take up a secondary structure with loops (7) of equal size and to form a polyhedral tertiary structure which it takes up at the implantation site during the implantation, wherein each face (2') of the polyhedron is formed by a large loop (7), wherein the polyhedron has one or several further smaller loops (8) which are smaller than the large loops (7) which form the faces (2') of the polyhedron, **characterised in that** the further smaller loops (8) are disposed on one or several faces (2'), edges (3') and/or corners (4') of the polyhedron.

2. Medical implant as claimed in Claim 1, **characterised in that** the polyhedron is a tetrahedron (6), an octahedron, a dodecahedron, in particular a pentagon dodecahedron, or an icosahedron.

3. Medical implant as claimed in any one of the preceding claims, **characterised in that** the loops (7, 8) are crossed loops.

4. Medical implant as claimed in any one of the preceding claims, **characterised in that** the numerical ratio of the smaller loops (8) to the larger loops (7) is approximately 1:1.

5. Medical implant as claimed in Claim 4, **characterised in that** the numerical ratio of the smaller loops (8) to the larger loops (7) is 1:1 and the loops (7, 8) are disposed alternately in the linear extent of the coil (9).

6. Medical implant as claimed in Claim 5, **characterised in that** a smaller loop (8) is disposed in the polyhedron in each of the faces (2) of the polyhedron which are formed by the large loops (7).

7. Medical implant as claimed in any one of the preceding claims, **characterised in that** at least one smaller loop (8) is disposed in the polyhedron in each case between two of the adjoining large loops (7).

8. Medical implant as claimed in any one of the preceding claims, **characterised in that** at least one smaller loop (8) is disposed in the polyhedron in each case between three adjoining large loops (7).

9. Medical implant as claimed in any one of the preceding claims, **characterised in that** in the extended state the coil (9) has a length of 50 to 600, preferably 100 to 500 mm.

10. Medical implant as claimed in any one of the preceding claims, **characterised by** a microcoil (9) with a lumen which is closed at least towards the distal end.

11. Medical implant as claimed in any one of the preceding claims, **characterised in that** the coil (9) has an external diameter of 0.1 to 0.5 mm, preferably 0.2 to 0.35 mm and particularly preferably 0.24 to 0.28 mm.

12. Medical implant as claimed in any one of the preceding claims, **characterised in that** least one of the wires which form the coil (9) or the wire which forms the coil (9) is made from a platinum-iridium or platinum-tungsten alloy or a metal alloy with shape memory characteristics.

13. Medical implant as claimed in any one of the preceding claims, **characterised in that** the coil (9) in the longitudinal axis of a filamentary shaped element (16) is drawn from a metal alloy with shape memory characteristics.

14. Medical implant as claimed in any one of the preceding claims, **characterised in that** the coil (9) in the longitudinal axis of a filamentary shaped element (16) is drawn from a polymer or a metal wire without shape memory characteristics.

15. Medical implant as claimed in Claim 13 or 14, **characterised in that** the diameter of the filamentary shaped element (16) decreases towards the distal end.

16. Medical implant as claimed in Claim 15, **characterised in that** the diameter of the filamentary shaped element (16) at the distal end is 25 to 50% of the rest of the diameter of the shaped element (16).

17. Medical implant as claimed in Claim 15 or 16, **characterised in that** the filamentary shaped element (16) tapers continuously in the distal end region.

18. Medical implant as claimed in any one of the preceding claims with at least one detachment module with an electrolytically corrodible area which is disposed in the coil (9) proximally with respect to the part forming the polyhedron.

19. Apparatus for the implantation of implants (1') in body vessels and cavities with an implant (1') as claimed in any one of the preceding Claims 1 to 18 and an aid to insertion which is releasably connected to the proximal end (11) of the implant (1').

20. Apparatus as claimed in Claim 19 with a catheter, a voltage source and a cathode, in which the implant (1') serves as anode and is displaceable in the longitudinal direction in the catheter, wherein the connection of implant (1') and aid to insertion has an electrolytically corrodible area so that in contact with a body fluid the implant (1') can be detached by electrolytic processes.

21. Apparatus as claimed in either Claim 19 or Claim 20, **characterised in that** the aid to insertion is constructed as a guide wire.

22. Apparatus as claimed in Claim 21, **characterised in that** the guide wire is constructed at least in part as a coil or a spring.

23. Apparatus as claimed in any one of Claims 19 to 22, **characterised in that** the implant (1') and the aid to insertion are connected to one another by a detachment module which preferably has an electrolytically corrodible area.

24. Apparatus as claimed in any one of Claims 19 to 23, **characterised in that** the detachment module comprises a proximal coil and a distal coil as well as a segment which is located between them, wherein the coils are made from a material which is less susceptible to electrolytic corrosion than the material of the intermediate segment.

25. Apparatus as claimed in any one of Claims 19 to 24, **characterised in that** the detachment module with the implant (1') or the aid to insertion is releasably connected by welding, soldering, glueing or mechanical joining, in particular by non-positive or positive joining.

26. Apparatus as claimed in any one of Claims 19 to 25, **characterised in that** the aid to insertion is surrounded at least partially by an electrically insulating shrink-fit hose or an electrically insulating coating.

## Revendications

1. Implant médical se présentant sous la forme d'un enroulement **(9)** étiré en longueur, dans lequel au moins une partie de l'enroulement **(9)** est préformée pour adopter une structure secondaire comportant des boucles **(7)** de taille identique et pour réaliser une structure tertiaire en forme de polyèdre, qu'elle adopte sur le site d'implantation, pendant l'implantation, dans lequel chaque face **(2')** du polyèdre est formée d'une grosse boucle **(7)** ; le polyèdre présentant une ou plusieurs autres boucles **(8)** plus petites, de plus petites dimensions que les grosses boucles **(7)** formant les faces **(2')** du polyèdre, **caractérisé en ce que** les autres boucles **(8)**, plus petites, sont disposées sur une ou plusieurs faces **(2')**, arêtes **(3')** et/ou coins **(4')** du polyèdre.

2. Implant médical selon la revendication 1, **caractérisé en ce que** le polyèdre est un tétraèdre **(6),** un hexaèdre, un octaèdre, un dodécaèdre, en particulier un dodécaèdre à pentagones.

3. Implant médical selon l'une des revendications précédentes, **caractérisé en ce que** les boucles **(7, 8)** sont des boucles fermées.

4. Implant médical selon l'une des revendications précédentes, **caractérisé en ce que** le rapport numérique entre les petites **(8)** et les grosses **(7)** boucles est d'environ 1:1.

5. Implant médical selon la revendication 4, **caractérisé en ce que** le rapport numérique entre les petites **(8)** et les grosses **(7)** boucles est d'environ 1:1, et **en ce que** les boucles **(7, 8)** sont disposées de façon alternée, dans l'étendue linéaire de l'enroulement (9).

6. Implant médical selon la revendication 5, **caractérisé en ce que,** dans le polyèdre, une petite boucle **(8)** est disposée dans chacune des faces de polyèdre **(2)** formées par les grosses boucles **(7).**

7. Implant médical selon l'une des revendications précédentes, **caractérisé en ce que,** dans le polyèdre, au moins une petite boucle **(8)** est disposée chaque fois entre deux des grosses boucles **(7)** limitrophes.

8. Implant médical selon l'une des revendications précédentes, **caractérisé en ce que,** dans le polyèdre, au moins une petite boucle **(8)** est disposée chaque fois entre trois grosses boucles **(7)** limitrophes.

9. Implant médical selon l'une des revendications précédentes, **caractérisé en ce que** l'enroulement **(9)** présente, à l'état étiré, une longueur dans la fourchette de 50 à 600, de préférence de 100 à 500 mm.

10. Implant médical selon l'une des revendications précédentes, **caractérisé par** un micro-enroulement **(9)** comportant un lumen, fermé au moins à l'extrémité distale.

11. Implant médical selon l'une des revendications précédentes, **caractérisé en ce que** l'enroulement **(9)** présente un diamètre extérieur de 0,1 à 0,5 mm, de préférence de 0,2 à 0,35 mm et, de façon particulièrement préférée, de 0,24 à 0,28 mm.

12. Implant médical selon l'une des revendications précédentes, **caractérisé en ce qu'**au moins l'un des fils formant l'enroulement **(9),** ou le fil formant l'enroulement **(9),** est composé d'un alliage platine-iridium, ou d'un alliage platine-tungstène, ou d'un alliage métallique ayant des propriétés de mémoire de forme.

13. Implant médical selon l'une des revendications précédentes, **caractérisé en ce que,** dans l'axe longitudinal, l'enroulement **(9)** est traversé par un élément de forme **(16)** filamenteux, composé d'un alliage métallique ayant des propriétés de mémoire de forme.

14. Implant médical selon l'une des revendications précédentes, **caractérisé en ce que,** dans l'axe longitudinal, l'enroulement **(9)** est traversé par un élément de maintien **(16)** filamenteux, composé d'un polymère ou d'un fil métallique n'ayant pas de propriété de mémoire de forme.

15. Implant médical selon la revendication 13 ou 14, **caractérisé en ce que** le diamètre de l'élément de forme **(16)** filamenteux diminue lorsqu'on évolue vers l'extrémité distale.

16. Implant médical selon la revendication 15, **caractérisé en ce que,** à l'extrémité distale, le diamètre de l'élément de forme **(16)** filamenteux est de 25 à 50 % sur le restant de l'élément de forme **(16).**

17. Implant médical selon la revendication 15 ou 16, **caractérisé en ce que,** dans la zone d'extrémité distale, l'élément de forme **(16)** filamenteux s'effile de façon continue.

18. Implant médical selon l'une des revendications précédentes, ayant au moins un module de détachement avec un emplacement susceptible d'être corrodé par voie électrolytique, disposé dans l'enroulement **(9)**, en position proximale par rapport à la partie formant le polyèdre.

19. Dispositif d'implantation d'implants **(1')** dans des vaisseaux corporels et des espaces creux, avec un implant **(1')** selon l'une des revendications 1 à 18 précédentes, et avec une aide d'insertion reliée, de façon désolidarisable, à l'extrémité proximale **(11)** de l'implant **(1')**.

20. Dispositif selon la revendication 19, avec un cathéter, une source de tension électrique et une cathode, pour lequel l'implant **(1')** sert d'anode et est déplaçable en direction longitudinale dans le cathéter, la liaison entre l'implant **(1')** et l'aide à l'insertion présentant un emplacement susceptible d'être corrodé par voie électrolytique, de manière que l'implant **(1')**, en contact avec un liquide corporel, puisse être séparé grâce à des processus électrolytiques.

21. Dispositif selon l'une des revendications 19 ou 20, **caractérisé en ce que** l'aide à l'insertion est réalisée sous forme de fil de guidage.

22. Dispositif selon la revendication 21, **caractérisé en ce que** le fil de guidage est réalisé, au moins partiellement, sous forme d'enroulement ou de ressort.

23. Dispositif selon l'une des revendications 19 à 22, **caractérisé en ce que** l'implant **(1')** et l'aide à l'insertion sont reliés entre eux par un module de détachement, présentant de préférence un emplacement susceptible d'être corrodé par voie électrolytique.

24. Dispositif selon l'une des revendications 19 à 23, **caractérisé en ce que** le module de détachement comprend un enroulement proximal et un enroulement distal, ainsi qu'un segment intermédiaire, les enroulements étant composés d'un matériau moins susceptible à la corrosion électrolytique que celui du segment intermédiaire.

25. Dispositif selon l'une des revendications 19 à 24, **caractérisé en ce que** le module de détachement est relié, de façon indésolidarisable, à l'implant **(1')** ou à l'aide à l'insertion, par soudage, brasage, collage ou par jointoyage mécanique, en particulier par une liaison à interaction de forces ou à ajustement de formes.

26. Dispositif selon l'une des revendications 19 à 25, **caractérisé en ce que** l'aide à l'insertion est entourée au moins partiellement par un tuyau rétractable isolant de l'électricité, ou un revêtement isolant de l'électricité.
